# FASCICULE DE BREVET EUROPEEN

(11) **EP 3 880 079 B1**
(45) Date de publication et mention de la délivrance du brevet: **03.01.2024**
(21) Numéro de dépôt: 19816825.4
(22) Date de dépôt: 10.10.2019
(51) Int. Cl.: A61B 8/00

(54) **SONDE AVEC CHAMBRE DE REFROIDISSEMENT ET PROCÉDÉ DE FABRICATION D'UNE TELLE SONDE**
SONDE MIT EINER KÜHLKAMMER UND VERFAHREN ZUR HERSTELLUNG EINER SOLCHEN SONDE
PROBE HAVING A COOLING CHAMBER AND METHOD FOR MANUFACTURING SUCH A PROBE

(30) Priorité: 16.11.2018 FR 1871577
(43) Date de publication de la demande: 22.09.2021
(73) Titulaire: SUPERSONIC IMAGINE, 13290 Aix-en-Provence (FR)
(72) Inventeur: VALENTIN, Matthieu, 13007 MARSEILLE (FR); ROUSSEAU, Etienne, 13100 AIX EN PROVENCE (FR); GIRAL, Frédéric, 83470 POURCIEUX (FR)
(74) Mandataire: Plasseraud IP
(86) Numéro de dépôt international: PCT/FR2019/052412
(87) Numéro de publication internationale: WO 2020/099742

(56) Documents cités:
- EP-A1- 1 707 122
- WO-A1-2016/162855
- US-A- 5 560 362
- US-A1- 2012 060 610

## Description

### DOMAINE TECHNIQUE

La présente invention est relative aux sondes notamment celles pour ultrasons.

### ETAT DE LA TECHNIQUE ANTERIEURE

Plus particulièrement, l'invention concerne une sonde pouvant faire partie d'un système d'imagerie par ultrasons.

L'imagerie par ultrasons fonctionne en émettant des ondes ultrasonores dans un médium et en enregistrant les ondes ultrasonores reflétées par le medium.

À cette fin, une sonde est utilisée pour positionner les éléments émetteurs/récepteurs d'ondes au niveau de l'organe à imager. Les ondes sont créées par des actuateurs, par exemple de type piézoélectriques ou cMUT qui, en fonctionnement produisent de façon induite de la chaleur. Cette chaleur peut être fonction de la puissance des ondes émises. La demande US 2009/234230 montre un exemple de sonde émettant à haute fréquence.

Cette chaleur peut parfois atteindre une température qui peut être inconfortable pour le patient et/ou non conforme aux normes en vigueur pour les matériaux médicaux. De plus, une augmentation de la température dans la sonde peut réduire les capacités de celle-ci de manière temporaire ou permanente. Des mécanismes sont parfois prévus pour éviter que la sonde ne soit endommagée, par exemple, la sonde peut être bridée en puissance lorsqu'une certaine température est atteinte afin de protéger ses composants internes. Par exemple, la température seuil peut être entre 50 °C et 80°C. Les sondes peuvent aussi être programmées pour s'éteindre lorsque cette température seuil est atteinte, afin de permettre à la sonde de refroidir. En outre, cet échauffement peut limiter la cadence des séquences d'émission des ultrasons de la sonde pour réaliser des images.

WO 2016/162855 A1 divulgue une sonde (et le correspondant procédé de fabrication) pour ultrasons comportant, une enveloppe, des éléments émetteurs/récepteurs, une unité d'interface à l'intérieur de l'enveloppe, connectée aux éléments émetteurs/récepteurs, une chambre de refroidissement étanche, remplie d'un liquide caloporteur, et disposée à l'intérieur de l'enveloppe, l'unité d'interface se trouvant au moins en partie dans la chambre de refroidissement ou en contact avec celle-ci.

### EXPOSE DE L'INVENTION

La présente invention a pour but de perfectionner les sondes du type mentionné ci-dessous, afin d'éviter l'échauffement excessif de la sonde, voire son endommagement.

**L'invention se rapporte ainsi à une sonde** telle que définie dans la revendication 1 et les revendications dépendantes.

Grâce à la présence de la chambre de refroidissement, la température de la sonde en fonctionnement peut être réduite de manière efficace sans nécessiter d'arrêt de la sonde.

En fonctionnement, la pluralité d'éléments émetteurs et/ou récepteurs d'ondes acoustiques produisant de la chaleur, et le liquide caloporteur déplace cette chaleur en direction d'une deuxième extrémité de la sonde, la deuxième extrémité étant distale de la pluralité d'éléments émetteurs et/ou récepteurs d'ondes acoustiques.

Dans divers modes de réalisation de la sonde selon l'invention, on peut éventuellement avoir recours en outre à l'une et/ou à l'autre des dispositions suivantes.

Selon un aspect, le liquide caloporteur est diélectrique.

Selon un aspect, le liquide caloporteur est à changement de phases, et possède une température de transition fonction de la température résultant de la chaleur produite par l'élément émetteur et/ou récepteur d'ondes acoustiques en fonctionnement.

Selon un aspect, la température de transition du liquide caloporteur est entre la température ambiante et une température de 90 degrés Celsius.

Selon un aspect, la chambre de refroidissement est remplie du liquide caloporteur et d'un gaz.

Selon un aspect, la partie de la chambre de refroidissement remplie du liquide caloporteur représente au moins 5% d'un volume de la chambre de refroidissement.

Selon un aspect, un capteur de pression et/ou un capteur de température est à l'intérieur de la sonde.

Selon un aspect, un élément bloquant d'ondes acoustiques est disposé entre ledit au moins un élément émetteur et/ou récepteur d'ondes acoustiques et l'unité d'interface, la chambre de refroidissement étant en contact avec l'élément bloquant d'ondes.

Selon un aspect, un élément bloquant d'ondes acoustiques est disposé entre ledit au moins un élément émetteur et/ou récepteur d'ondes acoustiques et l'unité d'interface, la chambre de refroidissement étant définie par une face postérieure de l'élément bloquant d'ondes et par une paroi interne de l'enveloppe.

Selon un aspect, l'unité d'interface est entièrement contenue dans la chambre de refroidissement.

Selon un aspect, l'unité d'interface comporte une antenne.

Selon un aspect, l'intérieur de la sonde comprend une chambre sèche séparée de la chambre de refroidissement, la chambre de refroidissement étant proximale de la première extrémité de la sonde.

Selon un aspect, une partie de l'unité d'interface est disposée dans la chambre sèche.

Selon un aspect, la chambre de refroidissement étanche comporte au moins une partie flexible.

Selon un aspect, la chambre de refroidissement comprend deux poches flexibles au moins partiellement remplies du liquide caloporteur.

**L'invention se rapporte également à un procédé de fabrication d'une** sonde tel que définit dans la revendication 14 et les revendications dépendantes.

Dans divers modes de réalisation du procédé selon l'invention, on peut éventuellement avoir recours en outre à l'une et/ou à l'autre des dispositions suivantes.

Selon un aspect, on remplit la chambre de refroidissement du liquide caloporteur et d'un gaz.

Selon un aspect, les deux dernières étapes du procédé sont réalisées par les étapes successives suivantes :
- on place dans l'enveloppe une poche flexible au moins partiellement remplie du liquide caloporteur, et
- on ferme de manière étanche l'enveloppe pour former la chambre de refroidissement étanche.

### BREVE DESCRIPTION DES DESSINS

D'autres caractéristiques et avantages de l'invention apparaîtront au cours de la description suivante d'un de ses modes de réalisation, donné à titre d'exemple non limitatif, en regard des dessins joints.

Sur les dessins :
- la figure 1a est une section de coupe frontale d'une sonde selon un premier mode de réalisation ;
- la figure 1b est une vue de côté d'une unité d'interface d'une sonde ;
- la figure 2 est une section de coupe frontale d'une sonde selon un deuxième mode de réalisation ;
- la figure 3 est une section de coupe frontale d'une sonde selon un troisième mode de réalisation ;
- la figure 4 est une section de coupe frontale d'une sonde selon un quatrième mode de réalisation ; et
- la figure 5 est une section de coupe latérale d'une sonde selon un cinquième mode de réalisation.

Sur les différentes figures, les mêmes références numériques désignent des éléments identiques ou similaires.

### DESCRIPTION DETAILLEE

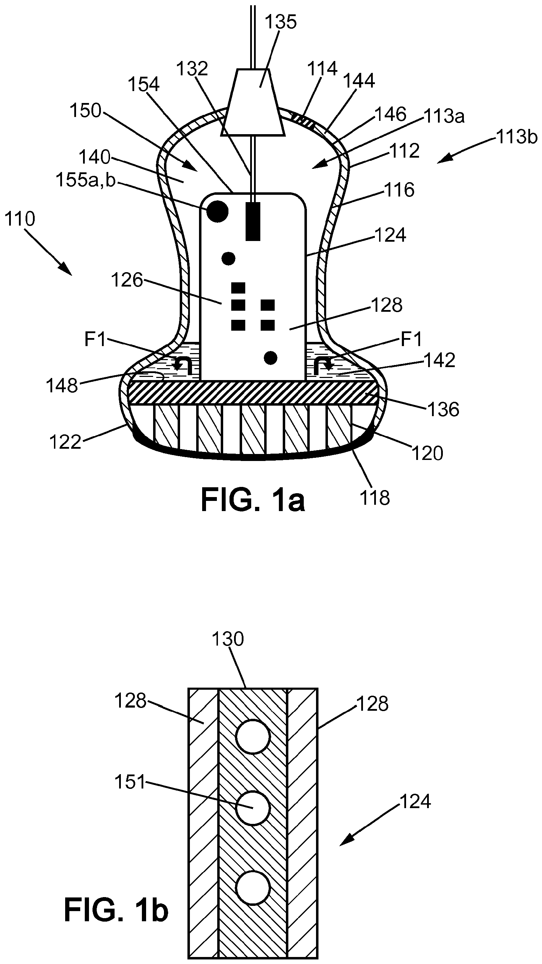

En référence à la **figure 1a** une **sonde 110,** notamment pour ondes acoustiques, par exemple ultrasons, va être décrite. La sonde 110 fait partie, dans un mode de réalisation, d'un système d'imagerie par ultrasons. Ce système peut par exemple être utilisé dans un contexte médical pour la visualisation d'organes et/ou de tissus. À cette fin, la sonde 110 permet l'émission et/ou la réception d'ondes ultrasons.

La sonde 110 inclut une **enveloppe 112** qui est une coque renfermant les différents composants de la sonde 110. L'enveloppe 112 délimite un **intérieur 113a** d'un **extérieur 113b** de la sonde 110. L'enveloppe 112 est par exemple globalement rigide pour faciliter la manipulation de la sonde 110. L'enveloppe 112 inclut une **portion de préhension 116,** globalement ergonomique, par laquelle un utilisateur manipule la sonde à une main. L'enveloppe 112 est composée de plus, d'une **surface d'émission et/ou réception 118** qui est adaptée à être en contact avec un tissu, par exemple la peau, ou bien un médium transmetteur d'ondes, comme par exemple un gel à ultrasons. La surface d'émission et/ou réception 118 est montrée dans les figures comme étant généralement plate, voire avec une légère courbure, planaire ou en 2D. La surface d'émission et/ou réception 118 peut toutefois présenter diverses formes en 3D, avec possiblement une courbure prononcée. L'enveloppe 112 est faite de un ou plusieurs matériaux isolants électriquement comme par exemple du plastique, par exemple du type ABS. L'enveloppe 112 peut être constituée de plusieurs parties assemblées. Par exemple l'enveloppe 112 (sans la surface d'émission et/ou réception 118) pourrait être constituée par une coque d'une seule pièce de plastique laissant une ouverture adaptée pour y recevoir la surface d'émission et/ou réception 118, ainsi connectée et fermant l'enveloppe 112. L'enveloppe 112 peut être rigide ou flexible, en totalité ou en partie. Selon un exemple, la surface d'émission et/ou réception 118 est constituée par un ou plusieurs polymère(s) flexible(s).

La sonde 110 inclut à l'intérieur 113a de l'enveloppe 112 une **pluralité d'éléments émetteurs et/ou récepteurs 120** d'ondes acoustiques disposés à une **première extrémité 122** de la sonde 110. Dans une variante, il est possible que la sonde 110 comprenne un seul élément émetteur et/ou récepteur 120, toutefois à des fins de simplification, ce terme sera employé au pluriel dans la description. La première extrémité 122 est une extrémité de la sonde 110 qui comprend la surface d'émission et/ou réception 118 de l'enveloppe 112. Selon un mode de réalisation, la pluralité d'éléments émetteurs et/ou récepteurs 120 comprend une pluralité d'éléments piézoélectriques (un seul élément piézo-électrique dans la variante présentée ci-dessus). La pluralité d'éléments émetteurs et/ou récepteurs 120 peut être disposée de façon à former une ligne ou front d'émission, ou bien une surface d'émission.

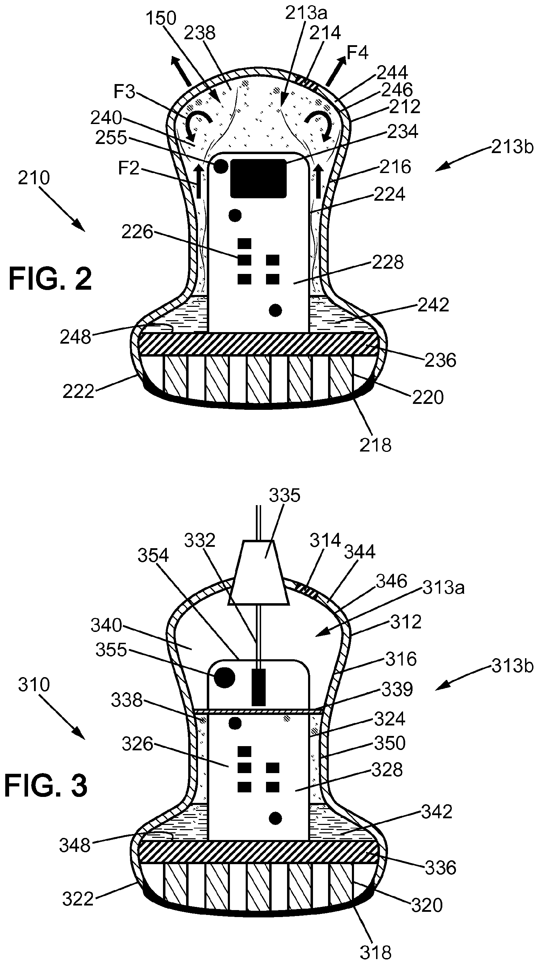

La pluralité d'éléments émetteurs et/ou récepteurs 120 est connectée à une **unité d'interface 124** qui se trouve à l'intérieur 113a de la sonde 110, de façon à transmettre des ordres d'émission et/ou à recevoir des données de réception et communiquer avec une unité de contrôle du système d'imagerie par ultrasons. L'unité d'interface 124, illustrée selon un mode de réalisation en figure 2, inclut par exemple deux **circuits imprimés 128,** chacun ayant un seul ou une **pluralité de composants électroniques 126,** actifs et /ou passifs, selon les modes de réalisation. De manière alternative, les circuits imprimés pourront être rigides ou flexibles. L'unité d'interface 124 peut comprendre plus ou moins de composants électroniques actifs (i.e. qui requièrent de l'énergie pour fonctionner), comme une batterie, un ou plusieurs pulseurs, une antenne et/ou un micro-processeur. Selon un mode de réalisation, l'unité d'interface 124 n'est composée que d'éléments passifs, de type connecteurs, et constitue un relai d'information avec l'unité de contrôle du système d'imagerie. Selon un autre mode de réalisation, l'unité d'interface 124 comprend des éléments actifs et participe au moins partiellement à l'élaboration d'ordres d'émission à destination de la pluralité d'éléments émetteurs et/ou récepteurs 120. Dans une variante, une partie de l'unité de contrôle du système d'imagerie peut être délocalisée au niveau de l'unité d'interface 124. Selon un autre mode de réalisation, l'unité d'interface 124 n'inclut pas de circuits électroniques actifs. Dans ce cas, l'unité d'interface est une structure permettant de regrouper des câbles coaxiaux et d'effectuer la connexion vers les éléments émetteurs et/ou récepteurs 120, par exemple par l'intermédiaire d'un ou plusieurs connecteurs et/ou de multiplexeurs.

Les circuits imprimés 128 sont dans ce mode de réalisation généralement séparés par un **drain thermique 130.** Le drain thermique 130 est convecteur de chaleur. Il est par exemple constitué d'une plaque d'aluminium. Selon un autre mode de réalisation, l'unité d'interface 124 n'a qu'un seul circuit imprimé, au moins deux circuits imprimés 128 et/ou n'a pas de drain thermique 130.

L'unité d'interface 124 communique avec un système d'imagerie par ultrasons (non représenté), dans un mode de réalisation, par l'intermédiaire d'une pluralité de **câbles coaxiaux 132.** Dans une variante, les câbles coaxiaux 132 sont regroupés dans une gaine étanche au liquide caloporteur 142. Par exemple, la gaine est réalisée en une résine, tel qu'un époxy. Dans une variante, la communication est sans fil et l'unité d'interface 124 peut comprendre une **antenne 234** comme illustré en figure 2. De ce fait, cette variante ne comporte a priori pas de câbles coaxiaux 132. Dans le mode de réalisation illustré par la figure 1a, les câbles coaxiaux 132 traversent l'enveloppe 112 via un **port étanche 135.** Dans le mode de réalisation de la figure 1a, le port étanche 135 se trouve à une **deuxième extrémité 144** de la sonde 110, la deuxième extrémité 144 étant distale de la pluralité d'éléments émetteurs et/ou récepteurs 120 d'ondes acoustiques. Le port étanche 135 par laquelle passent les tubes coaxiaux peut se trouver dans des variantes à un autre endroit de l'enveloppe 112.

L'unité d'interface 124 est séparée de la pluralité d'éléments émetteurs et/ou récepteurs 120 par un **élément bloquant d'ondes 136** connecté à la pluralité d'éléments émetteurs et/ou récepteurs 120. L'élément bloquant d'ondes 136 fournit un amortissement acoustique au moins partiel entre la pluralité d'éléments émetteurs et/ou récepteurs 120 et une partie de l'intérieur 113a de la sonde 110. En effet, lorsque la pluralité d'éléments émetteurs et/ou récepteurs 120 est en fonctionnement, les ondes acoustiques créées peuvent se propager vers l'intérieur 113a de la sonde 110 et rebondir sur les différents éléments se trouvant à l'intérieur de celle-ci, et ainsi créer un bruit acoustique indésirable. L'élément bloquant d'ondes 136 a pour but de bloquer une grande majorité de ces ondes se propageant vers l'intérieur 113a de la sonde 110, en les renvoyant ou bien en les absorbant. L'élément bloquant d'ondes 136 permet en outre de raccourcir la durée du pulse d'émission. L'élément bloquant d'ondes 136 est par exemple un élastomère, une résine souple ou une mousse composite. L'élément bloquant d'ondes 136 peut également ou alternativement être choisi pour être un élément conducteur de chaleur.

La sonde 110 inclut de plus une (ou plusieurs selon d'autres modes de réalisation) **chambre de refroidissement 140** pour refroidir la sonde 110 chauffée de façon induite par le fonctionnement de la pluralité d'éléments émetteurs et/ou récepteurs 120. La chambre de refroidissement 140 comprend un **liquide caloporteur 142** la remplissant au moins en partie. La chambre de refroidissement 140 est étanche aux fluides à l'intérieur de l'enveloppe 112, en particulier au liquide caloporteur et/ou au gaz si celui-ci est présent à l'intérieur 113a de l'enveloppe 112. La chambre de refroidissement peut ou non être confondue avec une partie de l'enveloppe 112. L'unité d'interface 124 se trouve au moins en partie dans la chambre de refroidissement 140 ou en contact avec la chambre de refroidissement 140. L'élément bloquant d'ondes 136 peut être en contact avec un extérieur de la chambre de refroidissement 140, ou en former une paroi. Plusieurs exemples non restrictifs et présentés à des fins d'illustration, de chambres de refroidissement 140 et leur disposition vis-à-vis de l'unité d'interface 124 et de l'enveloppe 112 sont par ailleurs illustrés en référence aux figures 2 à 6.

Dans le mode de réalisation de la figure 1a , la chambre de refroidissement 140 est confondue avec un intérieur 113a de l'enveloppe 112. La chambre de refroidissement 140 est donc délimitée par une **paroi interne 146** de l'enveloppe 112, 212 et par une **face postérieure 148** de l'élément bloquant d'ondes 136 (une face antérieure de l'élément bloquant d'ondes 136 étant en contact avec la pluralité d'éléments émetteurs et/ou récepteurs 120. L'élément bloquant d'ondes 136 est étanche, de sorte que le liquide caloporteur 142 ne pénètre pas la première extrémité 122 de la sonde 110 contenant la pluralité d'éléments émetteurs et/ou récepteurs 120. Selon d'autres alternatives, la chambre de refroidissement 140 peut ne pas se trouver en contact direct avec la face postérieure 148 de l'élément bloquant d'ondes 136. Un élément intermédiaire d'étanchéité pourrait par exemple s'y trouver.

En fonctionnement, la pluralité d'éléments émetteurs et/ou récepteurs 120 d'ondes acoustiques produit de la chaleur, et le liquide caloporteur 142 transfère une quantité de cette chaleur en direction de la deuxième extrémité 144 qui est une zone naturellement plus froide que la zone de la pluralité d'éléments émetteurs et/ou récepteurs 120, ce qui a pour effet de refroidir ainsi la sonde 110. Il est possible d'obtenir un refroidissement de la sonde 110 au moyen de diverses caractéristiques du liquide caloporteur 142. Par exemple, le refroidissement peut se faire par convection/conduction dans le liquide lui-même comme illustré en figure 1a ou bien par convection/conduction et changement de phase comme illustré en figure 2, les deux modes étant décrits ci-dessous.

Dans l'exemple illustré par la figure 1a, la chambre de refroidissement 140 est partiellement remplie du liquide caloporteur 142, de sorte qu'une autre partie de la chambre de refroidissement 140 est remplie d'un **gaz 150.** Le gaz 150 est un gaz compressible, par exemple de l'air. Selon un mode de réalisation, la chambre de refroidissement 140 est remplie du liquide caloporteur 142 convecteur de chaleur dans son intégralité. Selon un mode de réalisation, la chambre de refroidissement 140 est remplie en volume de liquide caloporteur 142 à 45% et de gaz 150 à 55%. Selon un mode de réalisation, la chambre de refroidissement 140 est remplie majoritairement de liquide caloporteur 142 et un reste du volume de la chambre de refroidissement 140 est rempli de gaz 150.

Selon un mode de réalisation, la chambre de refroidissement 140 est remplie en volume de liquide caloporteur 142 à au moins 5% et réciproquement du gaz 150 jusqu'à un maximum de 95%. Selon un mode de réalisation, la chambre de refroidissement 140 est remplie en volume de liquide caloporteur 142 à au moins 10% et réciproquement du gaz 150 jusqu'à un maximum de 90%. Le taux de remplissage peut dépendre de la nature du liquide caloporteur 142 et/ou du gaz 150 et/ou de la chambre de refroidissement 140 et/ou de tout élément de la sonde. Ce taux de remplissage pourra être optimisé par des tests.

Dans les modes de réalisations où la chambre de refroidissement 140 comporte du gaz 150, il se pourrait qu'une partie 114 de la chambre de refroidissement 140 (qui se pourrait être une partie de l'enveloppe 112 ou pas selon les modes de réalisation) soit flexible, de façon à servir de membrane de compensation. La membrane de compensation 114 peut être utilisée à chaque fois que la chambre de refroidissement n'est pas complètement remplie de liquide caloporteur 142. Dans certaines variantes, il est possible de se passer de la membrane de compensation 114 si les parois de la chambre de refroidissement (l'enveloppe 112 ou autre selon les modes de réalisation) présentent une flexibilité suffisante.

Dans l'exemple de la figure 1a, le liquide caloporteur 142 transfère tout ou partie de la chaleur de l'unité d'interface 124 et/ou de l'élément bloquant d'ondes 136 (donc indirectement de la pluralité d'éléments émetteurs et/ou récepteurs 120) en direction de la deuxième extrémité 144 par convection/convection, sans changement de phase. Les particules chauffées du liquide caloporteur 142 pendant le fonctionnement de la pluralité d'éléments émetteurs et/ou récepteurs 120 se déplacent en direction d'une zone du liquide caloporteur 142 plus froide, typiquement vers la zone du liquide caloporteur 142 la plus éloignée de la pluralité d'éléments émetteurs et/ou récepteurs 120. Lors de ce mouvement, les particules plus froides sont poussées en direction de la pluralité d'éléments émetteurs et/ou récepteurs 120. Elles se trouvent à leur tour alors chauffées par la chaleur dégagée par la pluralité d'éléments émetteurs et/ou récepteurs 120 en fonctionnement et un mouvement circulaire de réchauffement et refroidissement des particules du liquide caloporteur 142 est ainsi réalisé, comme illustré par les flèches F1. Le liquide caloporteur 142 est par exemple de l'eau, de l'huile, un alcool, un éther, un fluorocarbone, ou tout mélange de ces précédents composés.

Dans une variante, il se peut que l'échange de chaleur se fasse par conduction thermique, en l'absence ou présence de mouvement circulaire présenté ci-dessus. Ceci pourrait être le cas lorsque le liquide caloporteur 142 se présente sous forme plus ou moins visqueuse voire gélifiée. Ainsi, le liquide caloporteur 142 envisagé est de diverses viscosités, pouvant aller d'un liquide très peu visqueux à un liquide très visqueux voire un gel, à température ambiante. Le liquide caloporteur 142 est donc non solide et non gazeux dans une plage de fonctionnement normales d'utilisation. Par exemple, la température d'utilisation de la sonde peut varier entre 5°C et 35°C.

Dans une variante, il se peut que l'échange de chaleur se fasse par convection thermique. Dans ce cas, le liquide caloporteur 142 peut avoir avantageusement une viscosité comprise entre 0,32 cSt à 0,8 cSt pour favoriser ladite convection.

Selon un mode de réalisation, le liquide caloporteur 142 est diélectrique. Lorsque le liquide caloporteur 142 est diélectrique, l'unité d'interface 124 dans son ensemble peut se trouver dans la chambre de refroidissement 140, et notamment les parties de connexion 154 aux câbles coaxiaux 132 lorsqu'elles existent. Bien que le liquide caloporteur 142 soit illustré sur la figure 1a comme étant en contact avec une partie de l'unité d'interface 124, il est possible dans une variante que le liquide diélectrique soit en contact avec toute l'unité d'interface 124, de sorte que l'unité d'interface 124 soit submergée dans le liquide caloporteur 142, C'est le cas par exemple, lorsque la chambre de refroidissement 140 se trouve remplie exclusivement de liquide caloporteur 142. Selon un mode de réalisation, le liquide caloporteur 142 est le liquide 3M^{™} Novec 7000^{™}, ou un hydrofluoroether (HFE), ou encore un perfluorocarbure (PFC). Il est par exemple possible de diminuer la température de la sonde 110 de 3,5 degrés Celsius au bout de 45 min d'utilisation de la sonde 110 en utilisant comme liquide caloporteur 142 le liquide 3M^{™} Novec 7000^{™}.

On choisit de préférence un liquide caloporteur 142 dont la température d'ébullition est proche de la température interne de l'intérieur 113a.

On choisit de préférence un liquide caloporteur 142 de nature compatible avec les éléments de la sonde 110 dont il est en contact, de sorte à ne pas dégrader ces éléments-là.

Dans une variante, il est envisageable que l'unité d'interface 124 ait des **canaux de refroidissement 151,** traversant le drain thermique 130, comme illustré en figure 1b, permettant d'accroitre le refroidissement de l'unité d'interface 124. Selon ce mode de réalisation, le liquide caloporteur 142 s'écoule dans les canaux de refroidissement 151 traversant le drain thermique 130, favorisant ainsi l'échange thermique entre le liquide caloporteur 142 et l'unité d'interface 124. Les canaux de refroidissement 151 peuvent être de section et de nombre variés.

Selon un mode de réalisation, la sonde 110 comprend de plus un **capteur de pression 115a** dans la chambre de refroidissement 140, **et/ou un capteur de température 155b** qui peut se trouver dans la chambre de refroidissement 140 ou ailleurs à l'intérieur 113a de la sonde 110. Le capteur de pression 155a peut par exemple se trouver sur un des circuits imprimés 128, et le capteur de température 115b sur l'élément bloquant d'ondes 136. Le au moins un capteur de pression 115a et/ou le au moins un capteur de température 155b peuvent se trouver fonctionnellement reliés à au moins un des circuits imprimés 128 pour relayer l'information vers l'unité de contrôle du système d'ultrasons. Le capteur de pression 115a peut être utilisé lorsque la chambre de refroidissement 140 comporte le gaz 150, afin de détecter d'éventuelles fuites de gaz 150 ou de liquide 142 ou disfonctionnements du système. Le capteur de température 115b permet en particulier de détecter si la sonde 110 atteint une température supérieure à un seuil qui provoque une température de contact de la sonde avec la peau du patient supérieure à 43°C à +/- 3°C selon la norme IEC60601-1. En cas d'anomalie de fonctionnement de la sonde détectée par un ou plusieurs capteurs (par exemple fuite de liquide ou de gaz et/ou une température trop élevée), une alarme peut être activée, la sonde 110 pouvant alors être bridée dans son fonctionnement voire mise en arrêt. Selon un mode de réalisation, la sonde 110 comprend au moins un capteur de pression 115a et un capteur de température 115b qui, par la combinaison de leur information, peuvent être utilisés pour détecter d'éventuelles fuites de liquide caloporteur 142 lors du fonctionnement de la sonde 110.

Selon un autre mode de réalisation et tel qu'illustré à la figure 2, une sonde 210, peut avoir des caractéristiques et alternatives similaires à la sonde 110 du premier mode de réalisation. Cela inclut notamment l'utilisation d'un liquide caloporteur 242 à changement de phase liquide-gaz. Par concision, les caractéristiques communes des sondes 110 et 210 porteront les mêmes références numériques mais prises dans les deux centièmes et ne seront pas répétées ici. La sonde 210 diffère en particulier de la sonde 110 également en ce qu'elle ne contient pas de port étanche 135 ni de câbles coaxiaux 132, l'unité d'interface 224 communiquant avec l'unité de contrôle du système d'imagerie par ultrasons sans fil par l'intermédiaire de l'antenne 234.

Le liquide caloporteur 242 peut être choisi pour avoir une température d'ébullition (ou de transition) proche de la température locale à l'intérieur de la sonde 210 lorsque la pluralité d'éléments émetteurs et/ou récepteurs 220 est en fonctionnement. Par exemple, le liquide caloporteur 242 à changement de phases peut être sélectionné pour avoir une température d'ébullition proche d'une température locale de l'élément bloquant d'ondes 236 ou de l'unité d'interface 224. Selon un mode de réalisation, la température de transition du liquide caloporteur 242 est entre la température ambiante et 90°C (degrés Celsius), et en particulier de 34 degrés Celsius. Lorsque l'élément bloquant d'ondes 236 atteint la température de transition de phase, le liquide rentre en ébullition. Le **gaz 238** ainsi généré (qui peut être le gaz 250 ou un autre gaz) se déplace en direction de la deuxième extrémité 244 qui est naturellement plus froide que la zone de la sonde 210 où se trouve le liquide caloporteur 242 (flèche F2). Le contact avec cette zone plus froide condense le gaz 238 qui se transforme en gouttelettes de liquide caloporteur 242 et qui, rejoint la première extrémité 222 par écoulement liquide, rejoignant ainsi le liquide caloporteur 242 non encore évaporé (flèche F3). Une partie de la chaleur s'échappe optionnellement à travers l'enveloppe 212 par la deuxième extrémité 244 (flèche F4). Le gaz 250 présent dans la chambre de refroidissement 240 peut présenter les mêmes caractéristiques et alternatives que le gaz 150 décrit en référence avec la figure 1a.

En référence à la figure 3, un troisième mode de réalisation de la sonde 310 est similaire aux sondes 110 et 210, et possédant les alternatives présentées pour les sondes 110 et 210. Par concision, les caractéristiques communes des sondes 310 avec les sondes 110 et 210 porteront les mêmes références numériques mais dans les trois centièmes et ne seront pas répétées ici. Dans le mode de réalisation de la figure 3, la sonde 310 comprend de plus une chambre sèche 352. La chambre sèche 352 est séparée de manière étanche de la chambre de refroidissement 340 contenant le liquide caloporteur 342. Ce mode de réalisation peut être avantageusement utilisé afin d'éviter d'avoir une connexion 354 des câbles coaxiaux 332 située dans la chambre de refroidissement 340. Ainsi, dans ce mode de réalisation, la chambre de refroidissement 340 se trouve généralement vers la première extrémité 322, et la chambre sèche 352 vers la deuxième extrémité 344 de la sonde 310, la chambre sèche pouvant alors contenir une partie de l'unité d'interface qui contient les connexions 354 des câbles coaxiaux 332. L'unité d'interface 324 se trouve alors partiellement dans la chambre sèche 352 et partiellement dans la chambre de refroidissement 340, les deux chambres étant séparées par un **mur 339.** Dans l'exemple illustré en figure 3, seule la partie de l'unité d'interface 324 qui contient les connexions 354 aux câbles coaxiaux 332 se trouve dans la chambre sèche 352. Il est cependant possible qu'une plus grande partie de l'unité d'interface se trouve dans la chambre sèche 352. De manière similaire à l'exemple de la figure 2, il est possible dans des variantes que la sonde 310 n'ait pas de câbles coaxiaux 332 ni de port étanche 335.

Tel que discuté ci-dessus pour les figures 1a et 2, le liquide caloporteur 342 peut être à changement de phase ou pas, et remplir complètement ou partiellement la chambre de refroidissement 340. L'unité d'interface 324 étant en contact direct avec le liquide caloporteur 342, le liquide caloporteur 342 sera préférentiellement choisi pour être diélectrique.

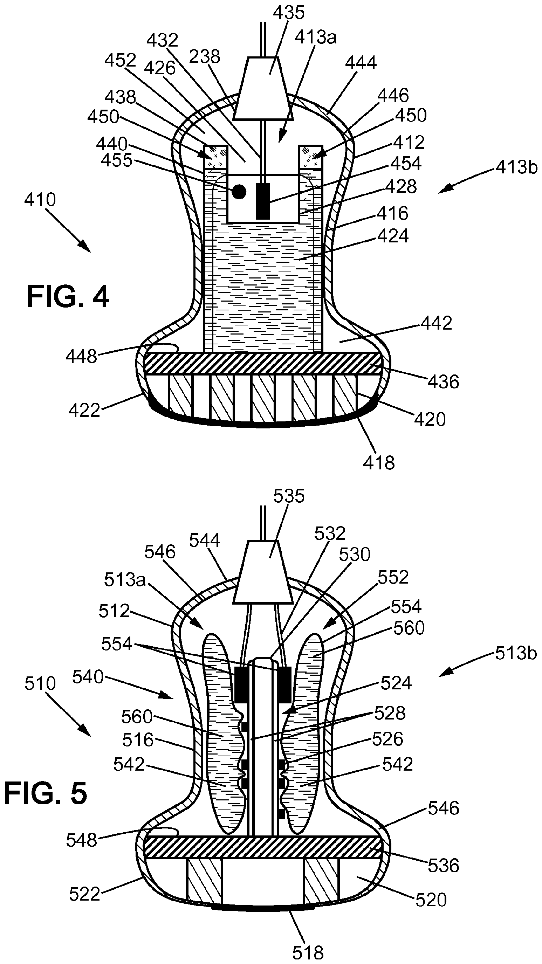

Concernant les figures 4 et 5, des modes de réalisation de la sonde montrant des chambres de refroidissement n'ayant pas l'enveloppe de la sonde comme paroi sont illustrées. Dans ces modes de réalisation, les parois de l'enveloppe délimitent une chambre sèche et la chambre de refroidissement est une chambre étanche contenue dans la chambre sèche.

Ainsi, en référence à la figure 4, une sonde 410 est similaire aux sondes 110, 210 et 310, et possède les alternatives des sondes 110, 210 et 310. Par concision, les caractéristiques communes de la sonde 410 avec les sondes 110, 210 et 310 porteront des références numériques similaires mais dans les quatre centièmes, et ne seront pas répétées ici. Dans ce mode de réalisation, la paroi interne 446 de l'enveloppe 412 définit la chambre sèche 452. La chambre de refroidissement 440 se trouve à l'intérieur de la chambre sèche 452. Une partie de la face postérieure 448 de l'élément bloquant d'ondes 436 définit une paroi de la chambre de refroidissement 440. Il est possible dans une variante que la chambre de refroidissement 440 ait une paroi qui soit en contact avec la face postérieure 448 de l'élément bloquant d'ondes 436. La chambre de refroidissement 440 est définie d'autre part par une paroi rigide 456 qui contourne les connexions 454 aux câbles coaxiaux 432. Il est possible dans une variante que la paroi 456 ne soit pas rigide et/ou que la paroi ne contourne pas les connexions 454 aux câbles coaxiaux 432. Dans ce dernier cas, le liquide caloporteur 452 est choisi pour être diélectrique. Il est possible dans une variante qu'en lieu et place d'une chambre de refroidissement 440, la sonde 410 ait deux ou plus chambres de refroidissement 440. Par exemple, la sonde 410 peut avoir une chambre de refroidissement 440 connectée à un coté de l'unité d'interface 424, similairement à ce qui est illustré pour le mode de réalisation de la figure 5.

La figure 5 illustre une variante du mode de réalisation de la figure 4. Une sonde 510 est similaire aux sondes 110, 210, 310 et 410, et possède les alternatives des sondes 110, 210, 310 et 410. La sonde 510 diffère de la sonde 410 en ce que la chambre de refroidissement 540 n'est pas définie en partie par la face postérieure 548 de l'élément bloquant d'ondes 536, mais par une paroi qui peut être ou pas en contact avec la face postérieure 548 de l'élément bloquant d'ondes 536. Ainsi, la chambre de refroidissement 540 comprend deux poches flexibles 560 en contact avec les circuits imprimés 528. Les poches 560 contiennent le liquide caloporteur 542 et sont étanches au liquide caloporteur 542 et/ou au gaz. Elles peuvent être remplies du liquide caloporteur 542 en totalité ou en partie, avec partiellement du gaz ou pas. Le liquide caloporteur 542 peut être à changement de phase ou pas, diélectrique ou pas, comme discuté dans les modes de réalisation précédents. La flexibilité permet avantageusement aux poches 560 d'avoir une plus grande surface de contact avec l'unité d'interface 524. Cependant, il est envisagé que les poches 560 ne soient pas flexibles. Il est aussi envisagé qu'il n'y ait qu'une poche flexible 560, ou plus de deux poches flexibles 560. Les poches flexibles peuvent par exemple être en contact avec la face postérieure 548 de l'élément bloquant d'ondes 536 ou pas, et les poches peuvent être de formes variées.

Eventuellement, dans ce mode de réalisation, l'enveloppe 512 n'est pas obligatoirement étanche étant donné que les poches 560 forment elles-mêmes des enveloppes étanches pour le liquide caloporteur 142.

La sonde de tous les précédents modes de réalisation peut être **fabriquée selon le procédé** explicité ci-dessous.

Notamment, dans ce procédé de fabrication :
- on fournit une enveloppe (112, 212, 312, 412, 512) pour définir un intérieur (113a, 213a, 313a, 413a, 513a) de la sonde (110, 210, 310, 410, 510),
- on fournit un ou plusieurs éléments émetteurs et/ou récepteurs d'ondes acoustiques (120, 220, 320, 420, 520) et on les dispose à une première extrémité (122, 222, 322, 422, 522) de l'enveloppe (110, 210, 310, 410, 510)
- on fournit une unité d'interface (124, 224, 324, 442, 524) et on la connecte à le ou les éléments émetteurs et/ou récepteurs (120, 220, 320, 420, 520), on place l'unité d'interface (124, 224, 324, 424, 524) à l'intérieur (113a, 213a, 313a, 413a, 513a) de l'enveloppe (112, 212, 312, 412, 512),
- on ferme de manière étanche l'enveloppe pour former une chambre de refroidissement (140, 240, 340, 440, 540) étanche et disposée à l'intérieur (113a, 213a, 313a, 413a, 513a) de l'enveloppe (112, 212, 312, 412, 512), l'unité d'interface (124, 224, 324, 424, 524) se trouvant au moins en partie dans la chambre de refroidissement (140, 240, 340, 440, 540) ou en contact avec la chambre de refroidissement (140, 240, 340, 440, 540), et
- on remplit au moins en partie la chambre de refroidissement (140, 240, 340, 440, 540) d'un liquide caloporteur (142, 242, 342, 442, 542).

Le remplissage de la chambre de refroidissement par le liquide caloporteur peut être effectué par tout moyen. Par exemple, on peut utiliser une seringue qui traverse une membrane élastique sur l'enveloppe, ou une alimentation en liquide caloporteur qui remplit la chambre de refroidissement par une valve pouvant être refermée.

Eventuellement, on remplit la chambre de refroidissement (140, 240, 340, 440, 540) du liquide caloporteur (142, 242, 342, 442, 542) mais aussi d'un gaz (150, 250, 350, 450). Ce remplissage du gaz peut être effectué simultanément, avant ou après le remplissage par le liquide caloporteur. Ce remplissage du gaz peu reprendre les moyens de remplissage par le liquide caloporteur ou avoir ses propres moyens de remplissage, c'est-à-dire un moyen séparé.

En outre, l'enveloppe peut être réalisée en plusieurs parties, et par exemple en deux parties. L'enveloppe peut alors être fermée de manière étanche en jointant lesdites deux parties, par exemple avec un moyen de collage et/ou d'étanchéité.

En outre, comme représenté en figure 5, une ou deux poches flexibles 560 sont utilisées. Dans ce cas, les deux dernières étapes du procédé (i.e. les étapes de fermeture de l'enveloppe et de remplissage par le liquide caloporteur) sont réalisées par les étapes successives suivantes :
- on place dans l'enveloppe 512 au moins une poche flexible 560 au moins partiellement remplie (préalablement remplie) du liquide caloporteur 542, et
- on ferme de manière étanche l'enveloppe pour former la chambre de refroidissement 540 étanche.

La ou les poches 560 se retrouvent ainsi enfermées à l'intérieur de l'enveloppe dans ladite chambre de refroidissement de la sonde.

## Revendications

1. **Sonde** (110, 210, 310, 410, 510), notamment pour ultrasons, comportant :
- une enveloppe (112, 212, 312, 412, 512) définissant un intérieur (113a, 213a, 313a, 413a, 513a) de la sonde (110, 210, 310, 410, 510),
- un ou plusieurs éléments émetteurs et/ou récepteurs (120, 220, 320, 420, 520) d'ondes acoustiques disposés à une première extrémité (122, 222, 322, 422, 522) de la sonde (110, 210, 310, 410, 510),
- une unité d'interface (124, 224, 324, 442, 524) connectée à l'élément émetteur et/ou récepteur (120, 220, 320, 420, 520), l'unité d'interface (124, 224, 324, 424, 524) se trouvant à l'intérieur (113a, 213a, 313a, 413a, 513a) de l'enveloppe (112, 212, 312, 412, 512),
- une chambre de refroidissement (140, 240, 340, 440, 540) étanche et disposée à l'intérieur (113a, 213a, 313a, 413a, 513a) de l'enveloppe (112, 212, 312, 412, 512), l'unité d'interface (124, 224, 324, 424, 524) se trouvant au moins en partie dans la chambre de refroidissement (140, 240, 340, 440, 540) ou en contact avec la chambre de refroidissement (140, 240, 340, 440, 540), la chambre de refroidissement (140, 240, 340, 440, 540) étant au moins en partie remplie d'un liquide caloporteur (142, 242, 342, 442, 542) ;
**caractérisée en ce que** la sonde comprend en outre
- un élément bloquant d'ondes acoustiques (136, 236, 336, 436, 536) disposé entre ledit au moins un élément émetteur et/ou récepteur (120, 220, 320, 420, 520) d'ondes acoustiques et l'unité d'interface (124, 224, 324, 442, 524), la chambre de refroidissement (140, 240, 340, 440, 540) étant en contact avec l'élément bloquant d'ondes (136, 236, 336, 436, 536), ou la chambre de refroidissement (140, 240, 340, 440, 540) étant définie par une face postérieure (148, 248, 348, 448, 548) de l'élément bloquant d'ondes (136, 236, 336, 436, 536) et par une paroi interne (146, 246, 346, 446, 546) de l'enveloppe (112, 212, 312, 412, 512).

2. Sonde selon la revendication 1, dans laquelle le liquide caloporteur (142, 242, 342, 442, 542) est diélectrique.

3. Sonde selon l'une des revendications 1 ou 2, dans laquelle le liquide caloporteur (242, 342, 442, 542) est à changement de phases, et possède une température de transition fonction de la température résultant de la chaleur produite par l'élément émetteur et/ou récepteur (120, 220, 320, 420, 520) d'ondes acoustiques en fonctionnement.

4. Sonde selon la revendication 3, dans laquelle la température de transition du liquide caloporteur (242, 342, 442, 542) est entre la température ambiante et une température de 90 degrés Celsius.

5. Sonde selon l'une des revendications 1 à 4, dans laquelle la chambre de refroidissement (140, 240, 340, 440, 540) est remplie du liquide caloporteur (142, 242, 342, 442, 542) et d'un gaz (150, 250, 350, 450).

6. Sonde selon la revendication 1 à 5, dans laquelle la partie de la chambre de refroidissement (140, 240, 340, 440, 540) remplie du liquide caloporteur (142, 242, 342, 442, 542) représente au moins 5% d'un volume de la chambre de refroidissement (140, 240, 340, 440, 540).

7. Sonde selon l'une des revendications 1 à 6, comprenant un capteur de pression et/ou un capteur de température (155a,b, 255a,b, 355a,b, 455a,b) à l'intérieur (113a, 213a, 313a, 413a, 513a) de la sonde (110, 210, 310, 410, 510).

8. Sonde selon l'une des revendications 1 à 7, dans laquelle l'unité d'interface (124, 224, 324, 442, 524) est entièrement contenue dans la chambre de refroidissement (140, 240, 340, 440, 540).

9. Sonde selon l'une des revendications 1 à 8, dans laquelle l'unité d'interface (124, 224, 324, 442, 524) comporte une antenne (234).

10. Sonde selon l'une des revendications 1 à 9, dans laquelle l'intérieur (113a, 213a, 313a, 413a, 513a) de la sonde (110, 210, 310, 410, 510) comprend une chambre sèche (352, 452, 552) séparée de la chambre de refroidissement (140, 240, 340, 440, 540), la chambre de refroidissement (140, 240, 340, 440, 540) étant proximale de la première extrémité (122, 222, 322, 422, 522) de la sonde (110, 210, 310, 410, 510).

11. Sonde selon la revendication 10, dans laquelle une partie de l'unité d'interface (124, 224, 324, 424, 524) est disposée dans la chambre sèche (352, 452, 552).

12. Sonde selon l'une des revendications 1 à 11, dans laquelle la chambre de refroidissement (140, 240, 340, 440, 540) étanche comporte au moins une partie flexible (114, 214, 314).

13. Sonde selon l'une des revendications 1 à 12, dans laquelle la chambre de refroidissement (140, 240, 340, 440, 540) comprend deux poches flexibles (560) au moins partiellement remplies du liquide caloporteur (542).

14. **Procédé de de fabrication d'une sonde** (110, 210, 310, 410, 510), notamment pour ultrasons, dans lequel :
- on fournit une enveloppe (112, 212, 312, 412, 512) pour définir un intérieur (113a, 213a, 313a, 413a, 513a) de la sonde (110, 210, 310, 410, 510),
- on fournit un ou plusieurs éléments émetteurs et/ou récepteurs d'ondes acoustiques (120, 220, 320, 420, 520) et on les dispose à une première extrémité (122, 222, 322, 422, 522) de l'enveloppe (110, 210, 310, 410, 510),
- on fournit une unité d'interface (124, 224, 324, 442, 524) et on la connecte à le ou les éléments émetteurs et/ou récepteurs (120, 220, 320, 420, 520), on place l'unité d'interface (124, 224, 324, 424, 524) à l'intérieur (113a, 213a, 313a, 413a, 513a) de l'enveloppe (112, 212, 312, 412, 512),
- on fournit un élément bloquant d'ondes acoustiques (136, 236, 336, 436, 536) disposé entre ledit un ou plusieurs éléments émetteurs et/ou récepteurs (120, 220, 320, 420, 520) d'ondes acoustiques et l'unité d'interface (124, 224, 324, 442, 524),
- on ferme de manière étanche l'enveloppe pour former une chambre de refroidissement (140, 240, 340, 440, 540) étanche et disposée à l'intérieur (113a, 213a, 313a, 413a, 513a) de l'enveloppe (112, 212, 312, 412, 512), l'unité d'interface (124, 224, 324, 424, 524) se trouvant au moins en partie dans la chambre de refroidissement (140, 240, 340, 440, 540) ou en contact avec la chambre de refroidissement (140, 240, 340, 440, 540), la chambre de refroidissement (140, 240, 340, 440, 540) étant en contact avec l'élément bloquant d'ondes (136, 236, 336, 436, 536), ou la chambre de refroidissement (140, 240, 340, 440, 540) étant définie par une face postérieure (148, 248, 348, 448, 548) de l'élément bloquant d'ondes (136, 236, 336, 436, 536) et par une paroi interne (146, 246, 346, 446, 546) de l'enveloppe (112, 212, 312, 412, 512), et
- on remplit au moins en partie la chambre de refroidissement (140, 240, 340, 440, 540) d'un liquide caloporteur (142, 242, 342, 442, 542).

15. Procédé selon la revendication 14, dans lequel on remplit la chambre de refroidissement (140, 240, 340, 440, 540) du liquide caloporteur (142, 242, 342, 442, 542) et d'un gaz (150, 250, 350, 450).

16. Procédé selon la revendication 14 ou la revendication 15, dans lequel les deux dernières étapes du procédé sont réalisées par les étapes successives suivantes :
- on place dans l'enveloppe (512) une poche flexible (560) au moins partiellement remplie du liquide caloporteur (542),
- on ferme de manière étanche l'enveloppe (512) pour former la chambre de refroidissement (540) étanche.

## Patentansprüche

1. Sonde (110, 210, 310, 410, 510), insbesondere für Ultraschall, umfassend:
- ein Gehäuse (112, 212, 312, 412, 512), das ein Inneres (113a, 213a, 313a, 413a, 513a) der Sonde (110, 210, 310, 410, 510) begrenzt,
- ein oder mehrere Sende- und/oder Empfangselemente (120, 220, 320, 420, 520) für akustische Wellen, die an einem ersten Ende (122, 222, 322, 422, 522) der Sonde (110, 210, 310, 410, 510) angeordnet sind,
- eine Schnittstelleneinheit (124, 224, 324, 442, 524), die mit dem Sende- und/oder Empfangselement (120, 220, 320, 420, 520) verbunden ist, wobei sich die Schnittstelleneinheit (124, 224, 324, 424, 524) im Inneren (113a, 213a, 313a, 413a, 513a) des Gehäuses (112, 212, 312, 412, 512) befindet,
- eine abgedichtete und im Inneren (113a, 213a, 313a, 413a, 513a) des Gehäuses (112, 212, 312, 412, 512) angeordnete Kühlkammer (140, 240, 340, 440, 540), wobei sich die Schnittstelleneinheit (124, 224, 324, 424, 524) wenigstens teilweise in der Kühlkammer (140, 240, 340, 440, 540) befindet, 240, 340, 440, 540) oder in Kontakt mit der Kühlkammer (140, 240, 340, 440, 540) befindet, wobei die Kühlkammer (140, 240, 340, 440, 540) wenigstens teilweise mit einer Wärmetauschflüssigkeit (142, 242, 342, 442, 542) gefüllt ist;
**dadurch gekennzeichnet, dass** die Sonde ferner umfasst
- ein Schallwellenblockierelement (136, 236, 336, 436, 536), das zwischen dem wenigstens einen Schallwellensende- und/oder -empfangselement (120, 220, 320, 420, 520) und der Schnittstelleneinheit (124, 224, 324, 442, 524) angeordnet ist, wobei die Kühlkammer (140, 240, 340, 440, 540) in Kontakt mit dem Schallwellenblockierelement (136, 236, 336, 436, 536) ist, oder die Kühlkammer (140, 240, 340, 440, 540) durch eine hintere Fläche (148, 248, 348, 448, 548) des Schallwellenblockierelements (136, 236, 336, 436, 536) und durch eine innere Wand (146, 246, 346, 446, 546) des Gehäuses (112, 212, 312, 412, 512) begrenzt ist.

2. Sonde nach Anspruch 1, wobei die Wärmetauschflüssigkeit (142, 242, 342, 442, 542) dielektrisch ist.

3. Sonde nach einem der Ansprüche 1 oder 2, wobei die Wärmetauschflüssigkeit (242, 342, 442, 542) phasenwechselnd ist und eine Übergangstemperatur besitzt, die von der Temperatur abhängt, die sich aus der Wärme ergibt, die von dem im Betrieb befindlichen Sende- und/oder Empfangselement (120, 220, 320, 420, 520) für akustische Wellen erzeugt wird.

4. Sonde nach Anspruch 3, wobei die Übergangstemperatur der Wärmetauschflüssigkeit (242, 342, 442, 542) zwischen Umgebungstemperatur und 90 Grad Celsius liegt.

5. Sonde nach einem der Ansprüche 1 bis 4, wobei die Kühlkammer (140, 240, 340, 440, 540) mit der Wärmetauschflüssigkeit (142, 242, 342, 442, 542) und einem Gas (150, 250, 350, 450) gefüllt ist.

6. Sonde nach Anspruch 1 bis 5, wobei der Teil der Kühlkammer (140, 240, 340, 440, 540), der mit der Wärmetauschflüssigkeit (142, 242, 342, 442, 542) gefüllt ist, wenigstens 5 % eines Volumens der Kühlkammer (140, 240, 340, 440, 540) darstellt.

7. Sonde nach einem der Ansprüche 1 bis 6, umfassend einen Drucksensor und/oder einen Temperatursensor (155a,b, 255a,b, 355a,b, 455a,b) im Inneren (113a, 213a, 313a, 413a, 513a) der Sonde (110, 210, 310, 410, 510).

8. Sonde nach einem der Ansprüche 1 bis 7, wobei die Schnittstelleneinheit (124, 224, 324, 442, 524) vollständig in der Kühlkammer (140, 240, 340, 440, 540) enthalten ist.

9. Sonde nach einem der Ansprüche 1 bis 8, wobei die Schnittstelleneinheit (124, 224, 324, 442, 524) eine Antenne (234) aufweist.

10. Sonde nach einem der Ansprüche 1 bis 9, wobei das Innere (113a, 213a, 313a, 413a, 513a) der Sonde (110, 210, 310, 410, 510) eine Trockenkammer (352, 452, 552) umfasst, die von der Kühlkammer (140, 240, 340, 440, 540) getrennt ist, wobei die Kühlkammer (140, 240, 340, 440, 540) proximal zu dem ersten Ende (122, 222, 322, 422, 522) der Sonde (110, 210, 310, 410, 510) liegt.

11. Sonde nach Anspruch 10, wobei ein Teil der Schnittstelleneinheit (124, 224, 324, 424, 524) in der Trockenkammer (352, 452, 552) angeordnet ist.

12. Sonde nach einem der Ansprüche 1 bis 11, wobei die wasserdichte Kühlkammer (140, 240, 340, 440, 540) wenigstens einen flexiblen Teil (114, 214, 314) aufweist.

13. Sonde nach einem der Ansprüche 1 bis 12, wobei die Kühlkammer (140, 240, 340, 440, 540) zwei flexible Beutel (560) umfasst, die wenigstens teilweise mit der Wärmetauschflüssigkeit (542) gefüllt sind.

14. Verfahren zur Herstellung einer Sonde (110, 210, 310, 410, 510), insbesondere für Ultraschall, wobei:
- ein Gehäuse (112, 212, 312, 412, 512) zum Begrenzen eines Innenraums (113a, 213a, 313a, 413a, 513a) der Sonde (110, 210, 310, 410, 510) vorgesehen wird,
- ein oder mehrere, akustische Wellen aussendende und/oder empfangende, Elemente (120, 220, 320, 420, 520) vorgesehen und an einem ersten Ende (122, 222, 322, 422, 522) des Gehäuses (110, 210, 310, 410, 510) angeordnet werden,
- eine Schnittstelleneinheit (124, 224, 324, 442, 524) vorgesehen und mit dem/den Sende- und/oder Empfangselement(en) (120, 220, 320, 420, 520) verbunden wird und die Schnittstelleneinheit (124, 224, 324, 424, 524) im Inneren (113a, 213a, 313a, 413a, 513a) des Gehäuses (112, 212, 312, 412, 512) angeordnet wird,
- ein Schallwellenblockierelement (136, 236, 336, 436, 536) vorgesehen wird, das zwischen dem einen oder den mehreren, akustische Wellen aussendenden und/oder empfangenden, Elementen (120, 220, 320, 420, 520) und der Schnittstelleneinheit (124, 224, 324, 442, 524) angeordnet wird,
- das Gehäuse dicht verschlossen wird, um eine dichte Kühlkammer (140, 240, 340, 440, 540) zu bilden, die im Inneren (113a, 213a, 313a, 413a, 513a) des Gehäuses (112, 212, 312, 513a) angeordnet wird, 412, 512), wobei sich die Schnittstelleneinheit (124, 224, 324, 424, 524) wenigstens teilweise in der Kühlkammer (140, 240, 340, 440, 540) oder in Kontakt mit der Kühlkammer (140, 240, 340, 440, 540) befindet, wobei die Kühlkammer (140, 240, 340, 440, 540) in Kontakt mit dem Schallwellenblockierelement (136, 236, 336, 436, 536) steht, oder die Kühlkammer (140, 240, 340, 440, 540) durch eine Rückseite (148, 248, 348, 448, 548) des Schallwellenblockierelements(136, 236, 336, 436, 536) und durch eine Innenwand (146, 246, 346, 446, 546) des Gehäuses (112, 212, 312, 412, 512) begrenzt wird, und
- die Kühlkammer (140, 240, 340, 440, 540) wenigstens teilweise mit einer Wärmetauschflüssigkeit (142, 242, 342, 442, 542) gefüllt wird.

15. Verfahren nach Anspruch 14, wobei die Kühlkammer (140, 240, 340, 440, 540) mit der Wärmetauschflüssigkeit (142, 242, 342, 442, 542) und einem Gas (150, 250, 350, 450) gefüllt wird.

16. Verfahren nach Anspruch 14 oder Anspruch 15, wobei die letzten beiden Schritte des Verfahrens durch die folgenden aufeinanderfolgenden Schritte realisiert werden:
- ein flexibler Beutel (560), der wenigstens teilweise mit der Wärmetauschflüssigkeit (542) gefüllt ist, wird in dem Gehäuse (512) platziert,
- das Gehäuse (512) wird dicht verschlossen, um die dichte Kühlkammer (540) zu bilden.

## Claims

1. **Probe** (110, 210, 310, 410, 510), in particular for ultrasound, comprising:
- a casing (112, 212, 312, 412, 512) defining an interior (113a, 213a, 313a, 413a, 513a) of the probe (110, 210, 310, 410, 510),
- one or more emitting and/or receiving elements for acoustic waves (120, 220, 320, 420, 520), arranged at a first end (122, 222, 322, 422, 522) of the probe (110, 210, 310, 410, 510),
- an interface unit (124, 224, 324, 442, 524) connected to the emitting and/or receiving element (120, 220, 320, 420, 520), the interface unit (124, 224, 324, 424, 524) being located within the interior (113a, 213a, 313a, 413a, 513a) of the casing (112, 212, 312, 412, 512), **characterized in that** the probe (110, 210, 310, 410, 510) comprises:
a sealed cooling chamber (140, 240, 340, 440, 540) arranged within the interior (113a, 213a, 313a, 413a, 513a) of the casing (112, 212, 312, 412, 512), the interface unit (124, 224, 324, 424, 524) being arranged at least partially in the cooling chamber (140, 240, 340, 440, 540) or in contact with the cooling chamber (140, 240, 340, 440, 540), the cooling chamber (140, 240, 340, 440, 540) being at least partially filled with a heat transfer liquid (142, 242, 342, 442, 542);
an acoustic-wave-blocking element (136, 236, 336, 436, 536) arranged between the interface unit (124, 224, 324, 442, 524) and said at least one emitting and/or receiving element (120, 220, 320, 420, 520) for acoustic waves, the cooling chamber (140, 240, 340, 440, 540) being in contact with the wave-blocking element (136, 236, 336, 436, 536), or the cooling chamber (140, 240, 340, 440, 540) being defined by a rear face (148, 248, 348, 448, 548) of the wave-blocking element (136, 236, 336, 436, 536) and by an inner wall (146, 246, 346, 446, 546) of the casing (112, 212, 312, 412, 512).

2. Probe according to claim 1, wherein the heat transfer liquid (142, 242, 342, 442, 542) is dielectric.

3. Probe according to one of claims 1 or 2, wherein the heat transfer liquid (242, 342, 442, 542) is phase changing, and has a transition temperature that is a function of the temperature resulting from the heat produced by the emitting and/or receiving element (120, 220, 320, 420, 520) for acoustic waves during operation.

4. Probe according to claim 3, wherein the transition temperature of the heat transfer liquid (242, 342, 442, 542) is between room temperature and a temperature of 90 degrees Celsius.

5. Probe according to one of claims 1 to 4, wherein the cooling chamber (140, 240, 340, 440, 540) is filled with the heat transfer liquid (142, 242, 342, 442, 542) and a gas (150, 250, 350, 450).

6. Probe according to claims 1 to 5, wherein the portion of the cooling chamber (140, 240, 340, 440, 540) filled with the heat transfer liquid (142, 242, 342, 442, 542) represents at least 5% of the volume of the cooling chamber (140, 240, 340, 440, 540).

7. Probe according to one of claims 1 to 6, comprising a pressure sensor and/or a temperature sensor (155a,b, 255a,b, 355a,b, 455a,b) within the interior (113a, 213a, 313a, 413a, 513a) of the probe (110, 210, 310, 410, 510).

8. Probe according to one of claims 1 to 7, wherein the interface unit (124, 224, 324, 442, 524) is completely contained within the cooling chamber (140, 240, 340, 440, 540) .

9. Probe according to one of claims 1 to 8, wherein the interface unit (124, 224, 324, 442, 524) comprises an antenna (234).

10. Probe according to one of claims 1 to 9, wherein the interior (113a, 213a, 313a, 413a, 513a) of the probe (110, 210, 310, 410, 510) comprises a dry chamber (352, 452, 552) separate from the cooling chamber (140, 240, 340, 440, 540), the cooling chamber (140, 240, 340, 440, 540) being proximal to the first end (122, 222, 322, 422, 522) of the probe (110, 210, 310, 410, 510).

11. Probe according to claim 10, wherein a portion of the interface unit (124, 224, 324, 424, 524) is arranged in the dry chamber (352, 452, 552).

12. Probe according to one of claims 1 to 11, wherein the sealed cooling chamber (140, 240, 340, 440, 540) comprises at least one flexible portion (114, 214, 314).

13. Probe according to one of claims 1 to 12, wherein the cooling chamber (140, 240, 340, 440, 540) comprises two flexible pouches (560) at least partially filled with the heat transfer liquid (542).

14. **Method of manufacturing a probe** (110, 210, 310, 410, 510), in particular for ultrasound, wherein:
- a casing (112, 212, 312, 412, 512) is provided to define an interior (113a, 213a, 313a, 413a, 513a) of the probe (110, 210, 310, 410, 510),
- one or more emitting and/or receiving elements for acoustic waves (120, 220, 320, 420, 520) are provided and they are placed at a first end (122, 222, 322, 422, 522) of the casing (110, 210, 310, 410, 510),
- an interface unit (124, 224, 324, 442, 524) is provided and it is connected to the emitting and/or receiving element (s) (120, 220, 320, 420, 520), the interface unit (124, 224, 324, 424, 524) is placed within the interior (113a, 213a, 313a, 413a, 513a) of the casing (112, 212, 312, 412, 512),
- a wave-blocking element (136, 236, 336, 436, 536) is arranged between said at least one emitting and/or receiving element (120, 220, 320, 420, 520) for acoustic waves and the interface unit (124, 224, 324, 442, 524),
- the casing is sealed closed to form a sealed cooling chamber (140, 240, 340, 440, 540) arranged within the interior (113a, 213a, 313a, 413a, 513a) of the casing (112), 212, 312, 412, 512), the interface unit (124, 224, 324, 424, 524) being at least partially arranged in the cooling chamber (140, 240, 340, 440, 540) or in contact with the cooling chamber (140, 240, 340, 440, 540), the cooling chamber (140, 240, 340, 440, 540) being in contact with the wave-blocking element (136, 236, 336, 436, 536), or the cooling chamber (140, 240, 340, 440, 540) being defined by a rear face (148, 248, 348, 448, 548) of the wave-blocking element (136, 236, 336, 436, 536) and by an inner wall (146, 246, 346, 446, 546) of the casing (112, 212, 312, 412, 512), and
- the cooling chamber (140, 240, 340, 440, 540) is at least partially filled with a heat transfer liquid (142, 242, 342, 442, 542) .

15. Method according to claim 14, wherein the cooling chamber (140, 240, 340, 440, 540) is filled with the heat transfer liquid (142, 242, 342, 442, 542) and a gas (150, 250, 350, 450).

16. Method according to claim 14 or claim 15, wherein the last two steps of the method are carried out by the following successive steps:
- a flexible pouch (560) at least partially filled with the heat transfer liquid (542) is placed in the casing (512),
- the casing (512) is sealed closed to form the sealed cooling chamber (540).
